# EUROPEAN PATENT SPECIFICATION

(11) **EP 2 256 128 B1**
(45) Date of publication and mention of the grant of the patent: **07.12.2011**
(21) Application number: 09075237.9
(22) Date of filing: 22.05.2009
(51) Int. Cl.: C07J 41/00, C07J 1/00

(54) **Method for synthesis of dienogest from estrone-3-methylether**
Verfahren zur Synthese von Dienogest aus Estron-3-Methylether
Procédé de synthèse de diénogest à partir de estrone-3-méthylether

(43) Date of publication of application: 01.12.2010
(73) Proprietor: HEYL Chemisch-Pharmazeutische Fabrik GmbH und Co. KG, 14167 Berlin (DE)
(72) Inventor: Döhler, Thomas, 99423 Weimar (DE); Werner, Michael, 99510 Apolda (DE); Walther, Dirk-Detlef, 99510 Apolda (DE)
(74) Representative: Schulz Junghans

(56) References cited:
- EP-B1- 0 411 736
- WO-A-2007/066158
- US-A- 2 729 654
- US-A- 2 940 990
- FEDOROVA O I ET AL: "SYNTHESIS OF SOME DERIVATIVES OF 19 NORTESTOSTERONE FROM ESTRA-1 3 5 10-TRIENES" PHARMACEUTICAL CHEMISTRY JOURNAL (ENGLISH TRANSLATION OF KHIMIKO-FARMATSEVTICHESKII ZHURNAL), vol. 8, no. 8, 1974, pages 462-465, XP002555944 ISSN: 0091-150X
- P. DE RUGGIERI: "19-nor-Steroidi - Nota I. Sulla 17-cianidrina del 19-nor-androst-4-en-3,17-dione; un miglioramento alla sintesi dei 17.alpha.-omologhi del 19-nor-testosterone" GAZZETTA CHIMICA ITALIANA., vol. 87, 1957, pages 795-804, XP008115011 SOCIETA CHIMICA ITALIANA, ROME.
- J. Fried and J. A. Edwards: "Organic Reactions in Steroid Chemistry", 1972, Van Nostrand Reinhold Company, New York, USA ISBN: 9999046061 vol. I, pages 51-52, * page 51, column 52 *

## Description

### Description

The present invention relates to the synthesis of 17α-cyanomethyl-17β-hydroxy-estra-4,9-diene-3-one (formula I, in the following "dienogest"), from 3-methoxy-estra-1,3,5-trien-17-one (EME, formula II).

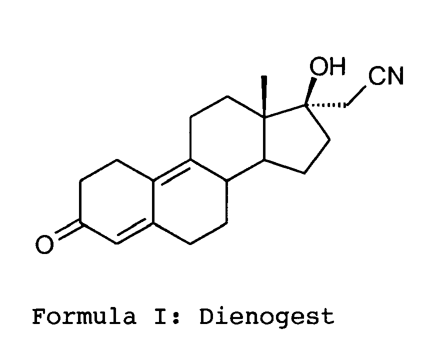

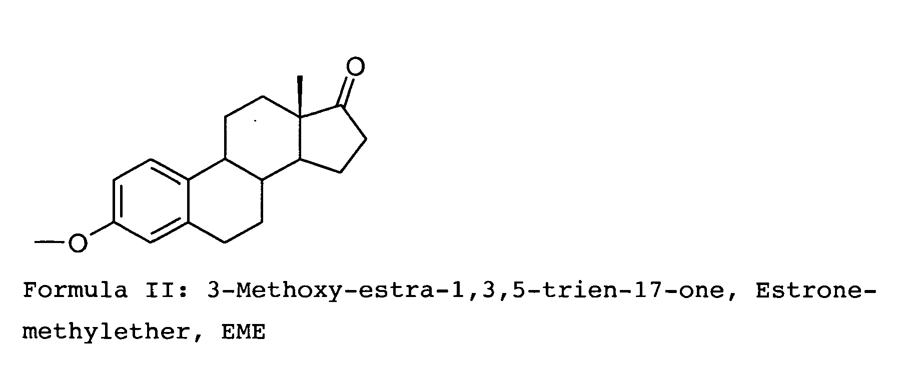

Dienogest is used primarily as a contraceptive in combination with ethinylestradiol.

DD132497, DD275247, W02007/066158 and EP1935898 describe synthetic methods to obtain dienogest.

The synthetic pathways known in the art suffer from disadvantages such as a high number of synthetic steps, comprising reactions that are difficult to increase in scale, such as the Oppenauer oxidation, or the use of undesirable reagents, such as cyanide, chromium oxidants or cerium.

The objective of the present invention is to provide a novel and economic way to obtain dienogest in high purity, from the cheap and readily available starting material, estrone-3-methylether (EME, formula II).

This objective is attained by the method of the present invention.

According to one embodiment of the present invention, dienogest is obtained from EME by a synthesis comprising the steps of:
a) reacting EME with alcohol in the presence of an acid in an organic solvent to form 3-methoxy-17,17-dialkoxy-estra-1,3,5-triene;
b) subjecting the product of step a) to partial reduction by reaction with alkali metal in liquid ammonia to form 3-methoxy-17,17-dialkoxy-estra-2,5(10)-diene;
c) treating the product of step b) with mild acid to form 3-methoxy-estra-2,5(10)-dien-17-one;
d) reacting the product of step c) with cyanomethyl lithium;
e) treating the product of step d) with oxalic acid;
f) treating the product of step e) with pyridinium tribromide in pyridine and
isolating dienogest as the product.

According to one embodiment of the present invention, the alcohol of step a) is a unbranched alkylic alcohol, preferably comprising one to three carbon atoms, preferably ethanol or methanol, most preferred methanol. The reaction may comprise the formation of a methylorthoformiate.

According to another embodiment of the present invention, the partial reduction of the steroid A ring of step b) is conducted at a temperature between minus 80°C and room temperature, preferably between minus 70 and minus 35 degrees Celsius, more preferred between minus 50 and minus 60 degrees, most preferred at minus 55 degrees Celsius. The preferred metals are sodium or lithium, most preferred is lithium.

According to another embodiment of the present invention, the partial reduction of the steroid A ring of step b) is conducted in a polar non-protic solvent, e.g. aliphatic, alicyclic ethers, and protic solvent, preferably alcohols, especially preferably isopropanol. The alcohol is added to the solution of the alkali metal, and the reaction is terminated by quenching with the same alcohol or another.

According to yet another embodiment of the present invention, step b) comprises the steps of providing liquid ammonia in a vessel under cooling, adding lithium and waiting for the appearance of blue colour; then providing the steroid component formed in step a) in an organic solvent, preferably using a mixture of tetrahydrofuran (THF) and isopropanole (i-prOH) as the solvent. Alternatively, according to yet another embodiment, said steroid component and ammonia may be provided, in an organic solvent, under cooling, and lithium may be added subsequently.

According to yet another embodiment of the present invention, the intermediate resulting from the Birch reduction reaction of step b) is quenched with alcohol to yield 3,17,17-trialkoxy-estra-2,5(10)-dien-17-one.

According to yet another embodiment of the present invention, the mild acid used in step c) is a diluted mineral acid or a weak organic acid. Diluted aqueous acetic acid is preferred.

According to yet another embodiment of the present invention, n-butyllithium is employed in formation of the cyanomethyllithium of step d). In general, the cyanomethyl lithium may be generated in situ by deprotonation of acetonitrile using strong base such as alkyl lithium, where alkyl can be C1-C6, lithium hexamethyldisilazide (LiHMDS) or lithium amides, e.g. lithium diisopropyl amide (LDA) or lithium 2,2,6,6-tetramethylpiperidide (LiTMP). LDA is preferred.

According to yet another embodiment, the cyanomethylation reaction of step d) is performed at a temperature between 0 degrees and minus 80 degrees, preferably between minus 20 degrees and minus 60 degrees and most preferably at minus 40 degrees Celsius. An embodiment is preferred where a solution of the steroid in an aliphatic or alicyclic ether, preferably THF, is added to a solution of cyanomethyl lithium. Alternatively, a solution of cyanomethyl lithium may be added to a solution of the steroid. According to one embodiment, 1 to 4 equivalents of cyanomethyl lithium, preferably 3 equivalents are used. The reaction may be terminated by quenching with water or alcohol.

According to yet another embodiment of the present invention, the reaction of step f) may be carried out in basic solvent, preferably pyridine, at lower temperature, preferably between 0°C and 5°C, where the solution of the oxidizing reagent is added to the solution of the steroid. Typically the reaction time is between 15 minutes and 6 hours, preferably 30 minutes. The process is characterized by dehydrohalogenation of the in situ formed bromo intermediate followed by acidic workup, preferably using sulphuric acid.

According to yet another embodiment of the present invention, the method of providing dienogest may comprise steps of isolating and purifying the intermediate product. These isolation and purification steps may be employed after any of the individual reactions of ketal formation, reduction of the steroid A ring to form the 2,5(10) diene, reconstitution of the 17-one by mild acid treatment, cyanomethylation, reconstitution of the 3-carbonyl and/or treatment by bromopyridine complex.

According to yet another embodiment of the present invention, the crude dienogest product of the method provided herein is subjected to a further purification step by at least one of the group consisting of recrystallization and chromatography.

According to yet another embodiment of the present invention, the solvent mixture employed in the chromatography step that may be added to obtain the final purified product makes use of a liquid phase comprising several, preferably three and at minimum two components with varying ratios. One of the components is an non-polar one, for example aromatic, aliphatic or cycloaliphatic hydrocarbon. Hetero-substituted hydrocarbons, such as halogenated hydrocarbons may be employed as well. Toluene or cyclohexane, especially dichloromethane, are preferred non-polar solvents. Preferred polar mixture component are alcohols, such as methanol and ethanol, esters and ketones, preferably ethyl acetate, acetone, as well as acetals, e.g. acetone dimethylacetal, formaldehyde dimethylacetal. 2-propanol is preferred in particular.

The ratio of polar and non-polar components may vary between 50:50 and 1:99, preferably between 65:98 and 85:96 and especially preferred 5:95.

According to yet another embodiment of the present invention, starting materials other than estrone-3-methylether may be employed. The 3-position may be functionalized by branched or unbranched alkyl, benzyl or may be esterified, e.g. by acetate.

The cyanomethylation reaction of step d) may proceed, according to a preferred embodiment of the present invention, at ambient to elevated temperatures for 15 minutes to 24 hours. An elevated temperature is preferred, the refluxing temperature of the solvent is preferred in particular. The reaction may be quenched by adding a base to the reaction mixture, e.g. aliphatic, cycloaliphatic and aromatic amines, which may be alkyl or aryl substituted. Amines may be primary, secondary and tertiary amines, where tertiary amines are preferred. Triethyl amine is especially preferred.

### Brief description of the Figures

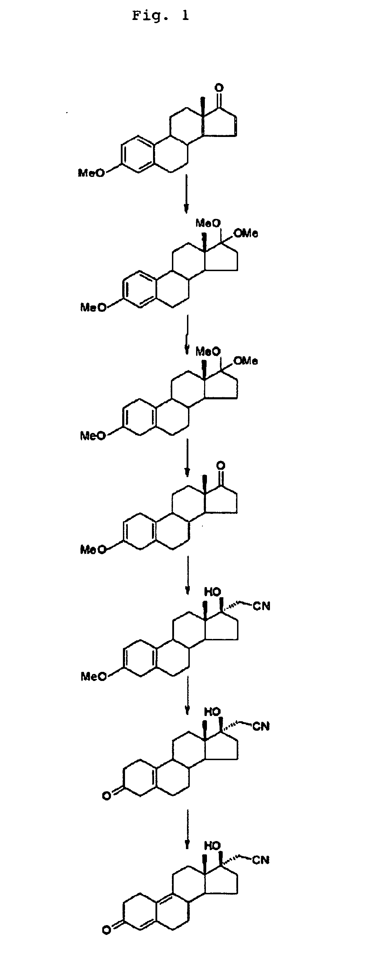

Fig. 1 shows the intermediate compounds of the process according to the embodiment comprising steps a) to f).

### Examples

### Example 1, 3,17,17-trimethoxy-estra-1,3,5-triene (estrone-3-methylether-methylketone, EMMK)

A suspension of estrone-3-methylether II (50.0 g, 176 mmol) in methanol was treated with methane sulfonic acid (0.55 ml, 0.81 g, 8 mmol). The reaction mixture was heated under reflux for 60 minutes. The completeness of the reaction was assessed by thin layer chromatography (TLC; toluene, chloroform, ethyl acetate, 6/3/1). The reaction was quenched by the addition of triethylamine (1.55 ml, 1.13 g, 11 mmol) and further stirring for 30 minutes. The reaction mixture was cooled to 0 °C, stirred for 30 minutes and filtered. The filtered solid was washed with water (1 1) extensively and dried *in vacuo.*

Yield: 55.4 g (167 mmol, 95 %)

HPLC: > 95%

### Example 2, 3,17,17-trimethoxy-estra-2,5(10)-diene

THF (200 ml) was cooled to -55 °C and liquid NH3 (300 ml) was added. Lithium (3.0 g, 428 mmol) was added under stirring. At -55 °C a solution of EMMK (example 1, 20 g, 61 mmol) and isopropanol (i-PrOH) (25 g) in THF (400 ml) was added dropwise. The temperature of the reaction mixture was held below -50 °C during addition. After complete addition, the mixture was stirred for another 60 minutes. The colour of the reaction mixture was monitored during addition and subsequent stirring; it is required to stay blue. Otherwise, lithium was added (1.0 g, 143 mmol) and the mixture was stirred again for 60 minutes. The reaction was quenched by adding i-PrOH (100 ml), heating the reaction mixture to -40°C and 30 minutes of stirring. NH3 was removed by heating the reaction mixture to minus 30°C. The reaction mixture was poured into 2 1 ice water. The resulting suspension was stirred for 30 minutes. The solid was filtered, washed with water (1 1). The isolated solid was used without further drying.

Yield: dried 19.0 g (57 mmol, 93 %)

HPLC: >95%

### Example 3, 3-methoxy-estra-2,5(10)-diene-17-one

The crude product of the reaction of example 2 (approximately 19.0 g, when dried, 57 mmol) was dissolved in THF (300 ml) and aqueous acetic acid (1 mol/l, 150 ml) was added. The resulting suspension was stirred at room temperature. The completeness of the reaction was confirmed by TLC (toluene, chloroform, ethyl acetate, 6/3/1). Ice water (500 ml) was added when the reaction was finished. The precipitated product was filtered, washed with water and dried *in vacuo.*

Yield: 16.0 g (56 mmol, 98 %)

HPLC: >92 %

### Example 4, 3-methoxy-17α-cyanomethyl-17β-hydroxy-estra-2,5(10)-diene (CMD)

Butyllithium (24.5, 245 mmol) was added to THF (250 ml) at minus 40°C. Acetonitrile (26.5 ml, 480 mmol) was added under stirring. A solution of dienon (example 3, 16.0 g, 56 mmol) in THF (300 ml) was added dropwise to the reaction mixture. After 90 minutes of stirring at minus 40°C the reaction was quenched by adding water (125 ml). The organic layer was removed in vacuo. Ice water (500 ml) was added to the remaining aqueous phase. The resulting precipitate was filtered and washed with water (500 ml). The isolated solid was used without further drying.

Yield: dried 18.7 g (55 mmol, 98 %)

HPLC: >90 %

### Example 5, 17α-cyanomethyl-17β-hydroxy-estra-5(10)-ene-3-one DCMK (from CMD)

The crude CMD (dried approximately 18.2 g, 55 mmol) was dissolved in THF (300 ml) and an aqueous solution of oxalic acid (13.5 g, 0.1 mol in 150 ml water) was added. The resulting suspension was stirred for 2 hours at room temperature. Within this time, the suspension turned into a clear solution. At complete conversion (controlled by TLC), ice water was added and the resulting precipitate was filtered, washed with water (500 ml) and dried in vacuo.

Yield: 14.8 g (47 mmol, 98 %)

HPLC: >85 %

### Example 6, Dienogest, crude

A solution of pyridinium tribromide (15.9 g) in pyridine (70 ml) was added dropwise to a cooled solution (0-5°C) of 5-DCMK (14.8 g, 47 mmol) in pyridine (90 ml). The reaction mixture was stirred for 30 minutes at 0-5°C. After this, the reaction mixture was heated to 70°C and this temperature was hold for 90 minutes. The reaction mixture was cooled to room temperature and poured into a mixture of sulphuric acid (45 ml) and water (200 g). The resulting precipitate is filtered, washed with water (250 ml) and dried in vacuo.

Yield: 13.1 g (42 mmol, 90 %)

HPLC: >90 %

### Example 7, Dienogest, pure

Dienogest, crude (example 6) is purified by HPLC using a column of 300 mm. The stationary phase is highly pure, spherical normal phase silica gel 10 µm with 6 nm pores. As non-polar phase, dicholoromethane, toluene and/or cyclohexane are used, polar mixture component is 2-propanol.

## Claims

1. Method for the synthesis of dienogest from 3-methoxy-estra-1,3,5-trien-17-one, comprising the steps of:
a) reacting 3-methoxy-estra-1,3,5-trien-17-one with alcohol in the presence of an acid in an organic solvent to form 3-methoxy-17,17-dialkoxy-estra-1,3,5-triene;
b) subjecting the product of step a) to partial reduction by reaction with alkali metal in liquid ammonia to form 3-methoxy-17,17-dialkoxy-estra-2,5(10)-diene;
c) treating the product of step b) with mild acid to form 3-methoxy-estra-2,5(10)-dien-17-one;
d) reacting the product of step c) with cyanomethyl lithium to form 3-methoxy-17α-cyanomethyl-17β-hydroxy-estra-2,5(10)-diene;
e) treating the product of step d) with oxalic acid to form 17α-cyanomethyl-17β-hydroxy-estr-5(10)-ene-3-one;
f) treating the product of step e) with pyridinium tribromide in pyridine and
isolating dienogest as the product.

2. Method according to claim 1, comprising isolating and purifying the intermediate product after one or more steps of the group consisting of step a), step b), step c), step d) and step e).

3. Method according at least one of claims 1 and claim 2, comprising subjecting the product dienogest to a further purification step by at least one of the group consisting of recrystallization and chromatography.

4. Method according to at least one of the previous claims, where the alcohol of step a) is methanol, yielding 3,17,17-trimethoxy-estra-1,3,5-triene as the product of step a).

5. Method according to at least one of the previous claims, where the alkali metal of step b) is lithium.

## Patentansprüche

1. Verfahren zur Herstellung von Dienogest aus 3-Methoxy-estra-1,3,5-trien-17-on, umfassend die Schritte:
a) Umsetzung von 3-Methoxy-estra-1,3,5-trien-17-on mit Alkohol in Anwesenheit einer Säure in einem organischen Lösungsmittel zu 3-Methoxy-17,17-dialkoxy-estra-1,3,5-trien;
b) teilweise Reduktion des Reaktionsprodukts des Schrittes a) durch Reaktion mit Alkalimetall in flüssigem Ammoniak zu 3-Methoxy-17,17-dialkoxy-estra-2,5(10)-dien;
c) Umsetzung des Reaktionsprodukts des Schrittes b) mit schwacher Säure zu 3-Methoxy-estra-2,5(10)-dien-17-on;
d) Umsetzung des Reaktionsproduktes des Schrittes c) mit CyanomethylLithium zu 3-Methoxy-17α-cyanomethyl-17β-hydroxy-estra-2,5(10)-dien;
e) Behandlung des Reaktionsproduktes des Schrittes d) mit Oxalsäure zu 17α-Cyanomethyl-17β-hydroxy-estr-5(10)-en-3-on;
f) Umsetzung des Reaktionsproduktes des Schrittes e) mit Pyridiniumtribromid in Pyridin und
Isolierung von Dienogest als Produkt.

2. Verfahren gemäß Anspruch 1, umfassend das Isolieren und Reinigen des Zwischenprodukts nach einem oder mehreren der Schritte der Gruppe bestehend aus Schritt a), Schritt b), Schritt c), Schritt d) und Schritt e).

3. Verfahren gemäß mindestens einem der Ansprüche 1 und 2, wobei das Produkt Dienogest einem weiteren Reinigungsschritt durch mindestens eine der Gruppe bestehend aus Rekristallisierung und Chromatographie unterworfen wird.

4. Verfahren gemäß mindestens einem der vorstehenden Ansprüche, wobei der Alkohol des Schrittes a) Methanol ist, was zu 3,17,17-Trimethoxy-estra-1,3,5-trien als Reaktionsprodukt des Schrittes a) führt.

5. Verfahren gemäß mindestens einem der vorstehenden Ansprüche, wobei das Alkalimetall des Schrittes b) Lithium ist.

## Revendications

1. Procédé de synthèse de diénogest à partir de 3-méthoxy-estra-1,3,5-triène-17-one, comprenant les étapes consistant à :
a) faire réagir 3-méthoxy-estra-1,3,5-triène-17-one avec de l'alcool en présence d'un acide dans un solvant organique pour former 3-méthoxy-17,17-dialkoxy-estra-1,3,5-triène ;
b) soumettre le produit de l'étape a) à une réduction partielle par réaction avec un métal alcalin dans de l'ammoniac liquide pour former 3-méthoxy-17,17-dialkoxy-estra-2,5(10)-diène ;
c) traiter le produit de l'étape b) avec un acide doux pour former 3-méthoxy-estra-2,5(10)-diène-17-one ;
d) faire réagir le produit de l'étape c) avec du cyanométhyl-lithium pour former 3-méthoxy-17α-cyanométhyl-17β-hydroxy-estra-2,5(10)-diène ;
e) traiter le produit de l'étape d) avec de l'acide oxalique pour former 17α-cyanométhyl-17β-hydroxy-estr-5(10)-ène-3-one ;
f) traiter le produit de l'étape e) avec du tribomure de pyridinium dans la pyridine et
isoler le diénogest en tant que produit.

2. Procédé selon la revendication 1, comprenant l'isolement et la purification du produit intermédiaire après une ou plusieurs étapes du groupe constitué de l'étape a), de l'étape b), de l'étape c), de l'étape d) et de l'étape e).

3. Procédé selon au moins une des revendications 1 et 2, comprenant la soumission du produit diénogest à une autre étape de purification par au moins un groupe constitué de la recristallisation et de la chromatographie.

4. Procédé selon au moins une des revendications précédentes, où l'alcool de l'étape a) est du méthanol, produisant 3,17,17-triméthoxy-estra-1,3,5-triène en tant que produit de l'étape a).

5. Procédé selon au moins une des revendications précédentes, où le métal alcalin de l'étape b) est du lithium.
